# EUROPEAN PATENT SPECIFICATION

(11) **EP 1 841 418 B1**
(45) Date of publication and mention of the grant of the patent: **17.04.2013**
(21) Application number: 06707702.4
(22) Date of filing: 11.01.2006
(51) Int. Cl.: A61K 31/198, A61P 25/30, A61P 25/32, A61P 25/34, A61P 25/36

(54) **Glycine reuptake inhibitors for treating drug and alcohol dependence**
Glycin-Rückbindungsinhibitoren zur Behandlung von Drogen- und Alkoholabhängigkeit
Inhibiteurs du recaptage de la glycine pour le traitement de la dépendance aux drogues et à l'alcool

(30) Priority: 14.01.2005 EP 05100218
(43) Date of publication of application: 10.10.2007
(73) Proprietor: MSD Oss B.V., 5349 AB Oss (NL)
(72) Inventor: MOLANDER, Anna, 405 30 Gotenborg (SE); SODERPALM, Bo, 413 45 Gotenborg (SE)
(74) Representative: Jaap, David Robert
(86) International application number: PCT/EP2006/050163
(87) International publication number: WO 2006/075011

(56) References cited:
- WO-A-00/07978
- WO-A-03/031435
- WO-A-2004/013100
- WO-A-2004/080454
- GB-A- 2 138 680

## Description

This invention relates to the Gly-T1 inhibitor N-Methyl-N-[(1R, 2S)-1,2,3,4-tetrahydro-6-methoxy-1-phenyl-2-naphthalenyl]methyl glycine for use in the treatment of alcohol addiction in humans.

Drug addiction, for example alcoholism, with its multitude of negative consequences to the afflicted, his/her relatives and the society remains a devastating disease for which there is a pressing need for new treatment principles. Indeed, during the last few years new pharmacological treatments such as naltrexone and acamprosate (Sass et al., 1996; Volpicelli et al., 1992; Berglund et al., 2003) have proven to be effective in the treatment of alcohol dependence. The effectiveness of these substances was predicted from studies using animal models of high alcohol consumption (Czachowski et al., 2001; Olive et al., 2002; Parkes and Sinclair, 2000). Unfortunately, naltrexone and acamprosate are, however, not effective in all alcoholics and new pharmacological treatments are therefore needed.

Reuptake of glycine via glycine transporter (GlyT) proteins into presynaptic nerve terminals or neighboring glial cells constitute an effective mechanism by which the postsynaptic action of glycine can be terminated and extracellular glycine levels returned to basal values. There are today two known types of glycine transporter proteins, the glial transporter (type 1), GlyT1, and the glycine neural transporter (type 2), GlyT2. The GlyT1 catalyses the removal of glycine from the synaptic deft and the GlyT2 is required for the reuptake and reloading of glycine into the synaptic vesicle (Gomeza et al., 2003; Curr Opin Drug Discov Devel 6(5): 675-82).

The present invention provides treatment or prevention of alcohol addiction in humans, comprising administering an effective amount of a GlyT1 inhibitor to a subject in need thereof.

The specific GlyT1 inhibitor used in the invention is N-methyl-N-[(1R,2S)-1,2,3,4-tetrahydm-6-methoxy-1-phenyl-2-naphthalenyl]methyl glycine (the free base of MTHMPNMglycine) or a pharmaceutically acceptable salt thereof. Also disclosed is 4-[3-fluoro-4-propoxyphenyl]-spiro[2H-1-benzopyran-2,4'-piperidine]-1'-acetic acid (the free base of FPPSBPAA) or a pharmaceutically acceptable salt thereof. These compounds are glycine reuptake type 1 inhibitors, which easily pass the brain blood barrier and have their main action on the GlyT1 protein with negligible action on the GlyT2 protein. For both compounds the HCl salts are prepared, which have the codes MTHMPNMglycine and FPPSBPAA, respectively. These compounds are known to be GlyT1 inhibitors and can be prepared by known methods. The preparation of MTHMPNMglycine as lithium salt is described in WO00/07978 (Gibson et al) and the preparation of FPPSBPAA is described in WO01/36423 (Gibson and Miller). Other useful GlyT inhibitors include SSR504734, ALX 5407, glycyldodecylamide, sarcosine, NFPS and other sarcosine analogues, CP-802,079, Org 24461 and Org 24598

Previously, such glycine transporter type 1 inhibitors have been suggested to find an application in the treatment of disorders such as epilepsy, and schizophrenia (Aragon and Lopez-Corcuera, 2003).

MTHMPNMglycine is a glycine reuptake inhibitor, which easily passes the brain blood barrier and has its main action on the GlyT1 protein with negligible action on the GlyT2 protein. If administered at 6 mg/kg i.p. to a rat weighing approx. 500 g this compound is expected to increase striatal extracellular glycine levels by approx. 50-70% for about 2.5 hours. In other experiments, oral administration of 13.5 mg/kg or more induced visible behavioral changes.

The anti-addiction effect can be maintained for a prolonged treatment period. This contrasts to the anti-addiction effect reported for several other substances, e.g. selective serotonin reuptake inhibitors, 5-HT_{1A} receptor agonists and opiate antagonists, which usually have prompt onsets of action on ethanol consumption but then lose effect after one or two weeks of treatment (Hedlund and Wahlstrom, 1996; Hedlund and Wahlstrom, 1998).

Our results indicate that increasing endogenous extracellular brain glycine levels by means of a selective GlyT1 inhibitor, for example MTHMPNMglycine, produces a robust, long-lasting and reversible decrease in ethanol intake and alcohol preference in a rat model with predictive value for the clinical situation.

The significant reduction of ethanol intake produced by the selective glycine reuptake inhibitors can be mediated via modulation of mesolimbic dopamine activity. Increased dopamine output in the nucleus accumbens of the rat (Blomqvist et al., 1993; Blomqvist et al., 1997; Di Chiara and Imperato, 1985; Imperato et al., 1986), as well as in man (Boileau et al., 2003) is related to the development and expression of drug addiction and alcoholism (Koob and Bloom, 1988; Robinson and Berridge, 1993; Wise, 1987; Wise and Rompre, 1989). Thus, while a GlyT1 inhibitor increases endogenous glycine levels this leads to an interference with glycine receptors in the nucleus accumbens and/or in the ventral tegmental area, producing disinhibition of mesolimbic DA neurons. This dopamine activation may in turn be associated with a decrease in ethanol consumption.

The treatment can be started and maintained during episodes of substance abuse. In previously published documents on glycine transport inhibitors and their possible utilities reference is made as entry in lists of a number of possible utilities to treatment of problems due to alcohol abuse or alcohol withdrawal (WO2004/013100), or, in combination with gamma vinyl GABA, to the treatment of convulsive and non-convulsive seizures associated with alcohol withdrawal (GB 2138 680). In none of these documents the teaching goes further than the metre mention of the option, without substantiating data or other incentives or instructions or evidence for the actual use of the mentioned options.

Glycine transport type 1 inhibitors are made available in the literature. Specifically, selective GlyT1 inhibitors are disclosed in WO00/07978 and WO01/36423 in which the methods of preparation of N-methyl-N-[(1R,2S)-1,2,3,4-tetrahydro-6-methoxy-1-phenyl-2-naphthalenyl]methylglycine (the free base of MTHMPNMglycine) and 4-[3-fluoro-4-propoxyphanyl]-spiro[2*H*-1-benzopyran-2,4'-piperidine]-1'-acetic acid (the free base of FPPSBPAA) can be found, respectively.

Compositions for administration to a subject in need of treatment for substance addiction problems can be prepared in accordance with standard techniques in the art of pharmaceutical sciences. The compounds can be used for humans in a dosage of 0.001-50 mg per kg body weight, preferably in a dosage of 0.01-20 mg per kg body weight, whereby the optimum dosage can be determined according to factors such as route of administration, desired duration of action, type of formulation (extended release versus immediate release) type of patient, type of compound required, efficacy of the compound and other physical characteristics of the recipient of the treatment, such co-morbidity of other diseases, liver metabolism capacity, etc. The treatment with a GlyT1 inhibitor can be combined with other drugs suitable in treatment of addictions. Since this is optional, the GlyT1 inhibitor can also be used as only active ingredient in a medicine for the treatment of substance addiction.

Selective transport inhibition and methods how to determine such a biological effect can be determined according to known techniques in the biochemistry of glycine. A specific method is described in the example below, on which basis a criterion pIC50 value of at least 6.0, or preferably 6.5, or even better 7.0 can be derived for clarity of the meaning of the term glycine transport type 1 inhibitor.

For diagnostic criteria for drug dependence, alcohol dependence etc. reference is made to the DSM IV revised edition.

### References

Aragon C, Lopez-Corcuera B (2003) Structure, function and regulation of glycine neurotransporters. Eur J Pharmacol 479(1-3):249-62.
Berglund M, Thelander S, Salaspuro M, Franck J, Andreasson S, Ojehagen A (2003) Treatment of alcohol abuse: an evidence-based review. Alcohol Clin Exp Res 27(10):1645-56.
Blomqvist O, Engel JA, Nissbrandt H, Soderpalm B (1993) The mesolimbic dopamine-activating properties of ethanol are antagonized by mecamylamine. Eur J Pharmacol 249(2):207-13.
Blomqvist O, Ericson M, Engel JA, Soderpalm B (1997) Accumbal dopamine overflow after ethanol: localization of the antagonizing effect of mecamylamine. Eur J Pharmacol 334(2-3):149-56.
Boileau I, Assaad JM, Pihl RO, Benkelfat C, Leyton M, Diksic M, Tremblay RE, Dagher A (2003) Alcohol promotes dopamine release in the human nucleus accumbens. Synapse 49(4):226-31.
Czachowski CL, Legg BH, Samson HH (2001) Effects of acamprosate on ethanol-seeking and self-administration in the rat. Alcohol Clin Exp Res 25(3):344-50.
Di Chiara G, Imperato A (1985) Ethanol preferentially stimulates dopamine release in the nucleus accumbens of freely moving rats. Eur J Pharmacol 115(1):131-2.
Fahlke C (1994) Alcohol Consumption in the Rat: Modulation by Adrenal Steroids and Mesotelencephalic Dopamine.
Gomeza J, Ohno K, Betz H (2003) Glycine transporter isoforms in the mammalian central nervous system: structures, functions and therapeutic promises. Curr Opin Drug Discov Devel 6(5):675-82.
Hedlund L, Wahlstrom G (1996) Buspirone as an inhibitor of voluntary ethanol intake in male rats. Alcohol Aloohol 31(2):149-56.
Hedlund L, Wahlstrom G (1998) Citalopram as an inhibitor of voluntary ethanol intake in the male rat. Alcohol 16(4):295-303.
Koob GF, Bloom FE (1988) Cellular and molecular mechanisms of drug dependence. Science 242(4879):715-23.
Olive MF, Nannini MA, Ou CJ, Koenig HN, Hodge CW (2002) Effects of acute acamprosate and homotaurine on ethanol intake and ethanol-stimulated mesolimbic dopamine release. Eur J Pharmacol 437(1-2):55-61.
Parks H, Sinclair JD (2000) Reduction of alcohol drinking and upregulation of opioid receptors by oral naltrexone in AA rats. Alcohol 21(3):215-21.
Robinson and Berridge, 1993 The neural basis of drug craving: an incentive-sensitization theory of addiction. Brain Research Rev. 18(3): 247-291
Sass H, Soyka M, Mann K, Ziegfgansberger W (1996) Relapse prevention by acamprosate. Results from a placebo-controlled study on alcohol dependence Arch Gen Psychiatry 53(8):673-80.
Volpicelli JR, Alterman Al, Hayashida M, O'Brien CP (1992) Naltrexone in the treatment of alcohol dependence. Arch Gen Psychiatry 49(11):876-80.
Wise RA (1987) The role of reward pathways in the development of drug dependence. Pharmacol Ther 35(1-2):227-63.
Wise RA, Rompre PP (1989) Brain dopamine and reward. Ann Rev Psychol 40(191):191-225.

### Examples

Method for determination of glycine uptake in CHO cells heterologously expressing the human GlyT-1b transporter.
**A**: Cloning: cDNA was generated by PCR according to the method described by Kim, K.-M. et al. Mol. Pharmacol. 1994, 45, 808-617. Sequence was verified by dideoxy sequencing using the ALF DNA sequencer™ (Pharmacia) and cloned into the expression construct pcDNA3 (Invitrogen).
**B**: Transfection: Transfection of hGlyT-1 b into CHO cells was performed using a standard calcium phosphate technique as described by Sambrook, J. et al. (1989) in Molecular Cloning: A Laboratory Manual, Cold Spring Harbor Laboratory, Cold Spring Harbor, NY.
**C**: Selection: Stably transfected cells were selected for 1 week in growth medium containing 1 mg.cm⁻³ Geneticin. Individual clones were picked for further analysis and positives passaged routinely as described below.
**D**: Culture conditions: Cells stably expressing the hGlyT-1b gene were cultured at 37 °C in a 5 % CO₂ atmosphere in DMEM - NUT.MIX. F12 with Glutamax-1 (Gibco) containing Geneticin (0.5mg.cm⁻³, Gibco) and supplemented with 10 % Fetalclone II (Hyclone). Maintenance culture was carried out in standard 80cm² ventilated flasks (2 x 10⁻⁶ m filter, Nunc) and cells were subcultured by trypsinisation (Sigma) when confluent.
**E**: Assay Procedure: Cells for uptake studies were plated in 96 well plates (17,000 cells per well) in the absence of Geneticin and cultured for 48 h before use. To measure glycine transport, cells were washed twice with Hanks' balanced salt solution (HBSS) prewarmed to 37 °C and excess fluid removed before addition of test compounds dissolved in 0.200 cm³ HBSS. Plates were incubated at 37 °C for 5 minutes before addition of [³H]glycine (0.050 cm³, 150 x 10⁻⁶ M, 248 Bq.nmol⁻¹, NEN) and incubation continued for a further 10 minutes. Uptake was terminated by washing cells with ice-cold HBSS before removal of excess fluid and addition of 0.200 cm³ scintillation cocktail to each well. Plates were sealed with adhesive film, shaken to ensure samples were homogenous before scintillation counting in a plate counter.
**F**: Data Analysis: Data were analysed using standard curve fitting procedures to produce a pIC₅₀ value for active compounds (where pIC₅₀ is the negative logarithm of the concentration of test compound causing 50 % inhibition of uptake).
**G**: Result:
   The pIC₅₀ values of compounds meant to be glycine transport type 1 inhibitors in this description are those having a pIC₅₀ value of at least 6.0.

### Effect on alcohol addiction in rats

### Materials and methods

### Animals

Male adult Wistar rats weighing 250-300g (approx. 50 days old) were supplied by Beekay (Stockholm, Sweden). The animals were housed in groups of four at a constant cage temperature (25°C) and humidity (65°C). The animals were allowed to adapt for one week to the novel environment before any experiment (Screening for ethanol preference) was performed. They were kept under regular light-dark conditions (light on at 07:00 a.m. and off at 19:00 p.m.) with free access to standard rat feed (Beekay feed) and tap water. This study was approved by the Ethics Committee for Animal Experiments, Göteborg, Sweden.

### Screening for ethanol preference

Rats had continuous access to a bottle of ethanol solution in addition to the water bottle. The ethanol concentration was gradually increased (2-4-6% v/v) over a two-week period. The animals were subsequently housed individually in plastic cages. They had continuous access to two bottles containing either tap water or 6% ethanol solution. Previous observations, using Wistar rats, indicate that the consumption of ethanol is maximal approx. at this concentration (Fahlke, 1994). Water and ethanol intake were measured over a 6-7-week period. The amount (g) of ethanol solution consumed, in percent of total fluid intake (g), was used as an index of ethanol preference. Rats were classified as low- (<20% ethanol), medium (20%-60% ethanol), or high- (>60% ethanol) preferring based on their ethanol preference.

### Drugs

Ethanol (AB Svensk sprit) was dissolved (2-4-6% v/v) with regular tap water and presented in regular plastic 300 ml bottles. MTHMPNMglycine, a glycine reuptake inhibitor with main action at GlyT1 (i.e. negligible action at GlyT2), was kindly provided by NV Organon and dissolved in NaCl (0.9%) and administered (i.p.). NaCl (0.9%) was used as vehicle.

### Experimental procedure

To adapt to the experimental procedure, approx. 80 days old ethanol high preferring male Wistar rats were limited to drink both ethanol (6% v/v) and water for only three hours/day during one week, so called limited access (limited access) drinking. Thereafter, daily MTHMPNMglycine or vehicle administration started. MTHMPNMglycine or vehicle was injected i.p. and the rats were allowed to rest for approx. 40 min after which they were presented a choice between ethanol and water and allowed to drink for 3 hours.
In the first experiment rats were challenged with MTHMPNMglycine (6 mg/kg) or vehicle for two weeks and alcohol and water intake were monitored. The experiment was preceded and ended by a limited access period.

In the second experiment a new set of rats was used in order to examine whether the effect of the glycine reuptake inhibitor could be reproduced. Again the rats were treated with MTHMPNMglycine (6 mg/kg) or vehicle for 13 days. In addition, the rats were thereafter exposed to a two-week period of alcohol deprivation (limited access), during which they were allowed to drink only water 3 hours per day. After the limited access period the rats were again challenged with MTHMPNMglycine or vehicle and re-exposed to ethanol for an additional time-period of 13 days. At the end of this second part of the experiment the dose of MTHMPNMglycine was gradually lowered (6-3-1.5 mg/kg), in order to examine whether the effect observed was dose-related.

The rats were presented to the water and ethanol (6% v/v) bottles approx. 40 minutes after drug administration. The choice of a drinking period of 3 hours was based on the information that if dosed i.p. at 6 mg/kg, MTHMPNMglycine is expected to increase striatal glycine levels by approx. 50-80% lasting about 2.5-3 hours.

### Statistics

The ethanol and water consumption data were statistically analysed using Analysis of variance (ANOVA) for repeated measures followed by post hoc analysis by means of Fisher's Protected Least Significant Difference (PLSD) test. Paired t-test was used for dependent comparisons between values obtained at different time-points. All values are expressed as means±SEM. A probability value (P) less than 0.05 was considered statistically significant.

### Results

### Experiment 1

MTHMPNMglycine significantly reduced ethanol intake (group effect [F(1,10)=9.239 p=0.0125], time effect [F(10,100)=1.765, p=0.0769] and interaction term [F(10,100)=0.721, p=0.7031], day 4-14, whereas there was no difference in ethanol intake between the groups during the limited access period (day 1-3) preceding the experiment as such.

There was no difference in water intake between the MTHMPNMglycine and vehicle treated groups (group effect [F(1,10)=0.028 p=0.8713], time effect [F(10,100)=3.031, p=0.0022] and interaction term [F(10,100)=1.164, p=0.3243], day 4-14).

The repeated measures ANOVA revealed no group effect [F(1,10)=1.978, p=0.1899], a time effect [F(10,100)=2.495, p=0.0102] but no interaction term [F(10,100)=0.764, p=0.6631], with respect to ethanol preference (day 4-14).

The total fluid intake was significantly reduced in the MTHMPNMglycine treated group, as compared to the vehicle treated group (group effect [F(1,10)=26.516, p=0.0004], time effect [F(10,100)=1.622, p=0.1110] and interaction term [F(10,100)=1.247, p=0.2710], day 4-14. However, also during the limited access period there was a significant difference in total fluid intake between the two groups (group effect [F(1,10)=6.254 p=0.0314], time effect [F(2,20)=1.813, p=0.1890] and interaction term [F(2,20)=0.751, p=0.4845], day 1-3.

### Experiment 2

A new set of male ethanol high-preferring Wistar rats were injected i.p. with either MTHMPNMglycine or vehicle. Approx. 40 minutes after injection all rats were exposed to a free choice between ethanol (6% v/v) and water for 3 hours. First all rats received MTHMPNMglycine (6 mg/kg) or vehicle daily during 13 days. This time-period was followed by 14 days of alcohol deprivation (limited access) when all rats were allowed to drink water only (3 hours/day). During the four last days of the limited access period the rats were again injected daily with either MTHMPNMglycine (6 mg/kg, i.p.) or vehicle, but were still allowed to drink only water. When the limited access period was completed the rats were again exposed to the choice between ethanol and water, starting approx. 40 min after the injection of MTHMPNMglycine (6 mg/kg) or vehicle. During the first 7 days (day 17-23) of this time-period the rats received 6 mg/kg of MTHMPNMglycine, the following 4 days (day 24-27) the dose was lowered to 3 mg/kg and finally the dose was lowered to 1.5 mg/kg for 2 days (day 28-29). The experiment was started and terminated by a limited access (limited access) period (3 days) when no drug or vehicle was administered and the rats were exposed to the free choice between ethanol (6% v/v) and water intake for 3 hours per day.

It was seen that ethanol intake was significantly reduced already during the first 6 days of MTHMPNMglycine treatment (group effect [F(1,13)=19.013 p=0.0008], time effect [F(5,65)=4.772, p=0.0009] and interaction term [F(5,65)=3.919, p=0.0036], day 4-10). This decrease was even more pronounced after one week of treatment (group effect [F(1,13)=29.362 p=0,0001], time effect [F(6,78)=12.809, p<0,0001] and interaction term [F(6,78)=3.131, p=0.0084], day 11-17). After two weeks of limited access the ethanol intake remained significantly reduced after MTHMPNMglycine (6 mg/kg), as compared to after vehicle (group effect [F(1,13)=5.554 p=0.0348], time effect [F(6,78)=1.954 p=0.0824] and interaction term [F(6,78)=0.582, p=0.7439], day 17-23). However, both groups significantly increased their ethanol intake after the alcohol deprivation period (MTHMPNMglycine day 14 vs. day 19, p=0.0135, paired t-test; vehicle day 14 vs. day 19 p=0.0006, paired t-test). After switching to 3 mg/kg of MTHMPNMglycine the difference between the groups was reduced (group effect [F(1,13)=1.179 p=0.2973], time effect [F(3,39)=6.092 p=0.0017] and interaction term [F(3,39)=1.398, p=0.2579], day 24-27), and this difference appeared totally abolished after lowering the dose to 1,5 mg/kg (group effect [F(1,13)=0.032 p=0.8614], time effect [F(1,13)=4.349 p=0.0573] and interaction term [F(1,13)=2.839, p=0.1158], day 28-29). As in the beginning of the experiment there was no difference in ethanol intake between the two groups during the last limited access period (day 30-32).

The water intake during MTHMPNMglycine treatment was, during the first part of the experiment (before limited access) not significantly different between the two groups. After the limited access period, MTHMPNMglycine treated rats showed a tendency to increase their water intake compared to the control rats (group effect [F(1,13)=4.032 p=0.0659], time effect [F(6,78)=3.328, p=0.0057] and interaction term [F(6,78)=1.031, p=0.4114], day 17-23). In the vehicle group there was no significant difference in water intake before limited access as compared to after limited access (p=0.0949, day 14 vs. day 19, paired t-test), whereas the MTHMPNMglycine treated rats significantly increased their water intake after the limited access period (p=0.0188, day 14 vs. day 19, paired t-test).

Ethanol preference did not differ between the MTHMPNMglycine and vehicle treated groups during the initial limited access period. Also during the first 6 days of MTHMPNMglycine (6 mg/kg) or vehicle treatment there was no significant difference between the two groups (group effect [F(1,13)=2.613 p=0.1300], time effect [F(5,65)=0.981, p=0.4360] and interaction term [F(5,65)=1.515, p=0.1974], day 4-10). After approx. one week of MTHMPNMglycine treatment a significant decrease in ethanol preference was observed in the MTHMPNMglycine treated rats (group effect [F(1,13)=35.038 p<0,0001], time effect [F(6,78)=1.495, p=0.1910] and interaction term [F(6,78)=0.583, p=0.7431], day 11-16). After two weeks of limited access (including 4 days of pre-treatment with MTHMPNMglycine or vehicle) the ethanol preference during MTHMPNMglycine treatment remained reduced, although not significantly, compared to that during vehicle treatment (group effect [F(1,13)=4.353 p=0.0572], time effect [F(6,78)=0.225 p=0.9676] and interaction term [F(6,78)=0.219, p=0.9695], day 17-23). Lowering of the MTHMPNMglycine dose to 3 mg/kg appeared to decrease the difference between the groups (group effect [F(1,13)=2.274 p=0.1555], time effect [F(3,39)=7.202 p=0.006] and interaction term [F(3,39)=1.825, p=1.1586], day 24-27), and this difference was totally abolished after lowering the MTHMPNMglycine dose to 1.5 mg/kg (group effect [F(1,13)=1.726 p=0.2117], time effect [F(1,13)=0.355 p=0.5618] and interaction term [F(1,13)=0.269, p=0.6127], day 28-29). As in the beginning of the experiment there was no difference between groups with respect to ethanol preference during the last limited access period (day 30-32).

After four days of MTHMPNMglycine treatment, the total fluid intake was significantly lowered as compared to that in the control group (group effect [F(1,13)=46.181 p<0.0001], time effect [F(8,104)=14.054, p<0.0001] and interaction term [F(8,104)=3.633, p=0.0009], day 8-16). Both vehicle and MTHMPNMglycine treated animals significantly increased their total fluid intake when comparing the intake during the last part of the limited access period with that after reintroducing a choice between ethanol and water (vehicle: day 14 vs. day 19, p<0.0001, paired t-test; MTHMPNMglycine: day 14 vs. day 19, p<0.0001, paired t-test). There was no difference in total fluid intake after the limited access period when comparing the two groups with each other.

### Discussion

This study demonstrates that a selective glycine reuptake inhibitor dose-dependently decreases voluntary ethanol intake in ethanol high preferring rats. The ethanol intake reducing effect of MTHMPNMglycine appeared to develop gradually and was in the second experiment most pronounced after 4-6 days of treatment. Thereafter the effect was maintained for the entire treatment period. Only the highest dose of MTHMPNMglycine (6 mg/kg) had a significant effect on ethanol intake. The lower doses (3 and 1.5 mg/kg) did not result in a significant reduction even if a slight decrease of the ethanol intake was observed after 3 mg/kg. The suppressive effect of MTHMPNMglycine on ethanol intake appeared robust, dose-related and reversible, indicating that the effect observed is specific and not due to chance fluctuations of ethanol intake.

In the second experiment, the MTHMPNMglycine (6 mg/kg) and vehicle treated rats were exposed to a 14-day period of alcohol deprivation (limited access). Ethanol intake was influenced by the limited access period, since both vehicle and MTHMPNMglycine treated animals clearly increased their ethanol intake after the limited access period. Interestingly, the ethanol intake in the MTHMPNMglycine treated group remained significantly decreased compared to that in the vehicle treated group also after the limited access period, even if the difference may not have been as pronounced as before. Somewhat unexpectedly the limited access period increased also water intake, particularly among MTHMPNMglycine treated rats, indicating that after a limited access period MTHMPNMglycine treated rats have a tendency to compensate for their reduced ethanol intake with water intake. Other explanations for this outcome may be that the rats have changed their fluid intake level during the limited access period due to some unspecific environmental change or that ethanol-induced dehydration has induced the increase in water intake.

Also ethanol preference was influenced by MTHMPNMglycine. In the first experiment the ethanol preference appeared to decrease in the MTHMPNMglycine treated rats, as compared to the vehicle treated group, although the difference did not reach statistical significance. In the second experiment the ethanol preference was significantly lower in the MTHMPNMglycine group compared to controls both before and after the limited access period. The limited access period failed to enhance ethanol preference in either group. The reason for this is probably that the rats used in the present study already were extremely ethanol high preferring (approx. 90%), and hence it is difficult to further increase ethanol preference in these animals.

There was no significant difference between MTHMPNMglycine treated rats and controls with respect to water intake, whereas in the first experiment and in the first part of the second experiment the total fluid intake was significantly lower in MTHMPNMglycine treated rats. This difference between groups was apparently mainly due to a decrease in ethanol intake, and was attenuated when water intake increased after the limited access period. Thus, a lowering of total fluid intake does not necessarily always occur during MTHMPNMglycine treatment. Taken together, these results indicate that the rats are probably not suffering from toxicological effects imposed by the compound. Also as judged from gross behavior, the animals were behaviorally unaffected by the doses of MTHMPNMglycine used in this study. In dose-finding pilot experiments MTHMPNMglycine in a dose of 10 mg/kg induced behavioral changes in one out of ten rats, lasting for approx 10-15 min. The MTHMPNMglycine treated rats had a significant lowered mean body weight during the limited access/experimental time compared to the vehicle treated rats.

## Claims

1. The Gly-T1 inhibitor *N*-methyl-*N*-[(1 R,2S)-1,2,3,4-tetrahydro-6-methoxy-1-phenyl-2-naphthalenyl]methyl glycine or a prharmaceutically acceptable salt thereof, for use in the treatment of alcohol addiction in humans.

2. Use of the Gly-T1 inhibitor *N*-methyl-*N*-[(1 R,2S)-1,2,3,4-tetrahydro-6-methoxy-1-phenyl-2-naphthalenyl] methyl glycine or a pharmaceutical acceptable salt thereof, for the manufacture of a medicament for the treatment of alcohol addiction in humans.

## Patentansprüche

1. Der Gly-T1-Inhibitor *N*-Methyl-*N*-[(1R,2S)-1,2,3,4-tetrahydro-6-methoxy-1-phenyl-2-naphthalinyl]methylglycin oder ein pharmazeutisch annehmbares Salz davon zur Verwendung bei der Behandlung von Alkoholsucht bei Menschen.

2. Verwendung des Gly-T1-Inhibitors *N*-Methyl-*N*-[(1R,2S)-1,2,3,4-tetrahydro-6-methoxy-1-phenyl-2-naphthalinyl]methylglycin oder eines pharmazeutisch annehmbaren Salzes davon zur Herstellung eines Medikaments zur Behandlung von Alkoholsucht bei Menschen.

## Revendications

1. L'inhibiteur de Gly-T1, la N-méthyl-N-[(1R,2S)-1,2,3,4-tétrahydro-6-méthoxy-1-phényl-2-naphtalényl]méthylglycine, ou un de ses sels pharmaceutiquement acceptables, destiné à être utilisé dans le traitement de l'alcoolisme chez l'être humain.

2. Utilisation de l'inhibiteur de Gly-T1, la N-méthyl-N-[(1R,2S)-1,2,3,4-tétrahydro-6-méthoxy-1-phényl-2-naphtalényl]méthylglycine, ou d'un de ses sels pharmaceutiquement acceptables, pour la fabrication d'un médicament destiné au traitement de l'alcoolisme chez l'être humain.
